# EUROPEAN PATENT APPLICATION

(11) **EP 4 505 995 A1**
(43) Date of publication of application: **12.02.2025**
(21) Application number: 24184662.5
(22) Date of filing: 26.06.2024
(51) Int. Cl.: A61K 9/00, A61K 9/08, A61K 31/4015, A61K 47/12, A61P 25/08

(54) **ORAL SOLUTION COMPOSITION COMPRISING BRIVARACETAM**

(30) Priority: 11.08.2023 TR 202309658
(71) Applicant: Sanovel Ilac Sanayi ve Ticaret A.S., Sisli - Istanbul (TR)
(72) Inventor: SUNEL, Fatih, Istanbul (TR); BILGEHAN ATAK, Fadime, Istanbul (TR); ATAMAN, Seval, Istanbul (TR); YAZICI, Ozlem, Istanbul (TR)
(74) Representative: Genç Ilhan, Oznur

(57) **Abstract**

The present invention relates to an oral solution composition comprising brivaracetam and at least one sweetening agent is sodium cyclamate, acesulfame potassium or mixtures thereof. Furthermore, the oral solution composition is also obtained by using an effective process which is simple, rapid, cost effective, time-saving and industrially convenient process.

## Description

### Field of the Invention

The present invention relates to an oral solution composition comprising brivaracetam and at least one sweetening agent is sodium cyclamate, acesulfame potassium or mixtures thereof. Furthermore, the oral solution composition is also obtained by using an effective process which is simple, rapid, cost effective, time-saving and industrially convenient process.

### Background of the Invention

The chemical name of brivaracetam is (2S)-2-[(4R)-2-oxo-4-propyl-pyrrolidin-1-yl]butanamide and its chemical structure is shown in the Formula I.

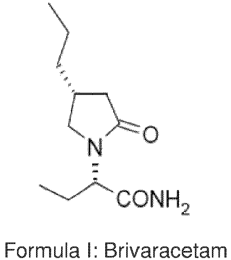

Brivaracetam is a white to off-white crystalline powder. It is very soluble in water, buffer (pH 1.2, 4.5, and 7.4), ethanol, methanol, and glacial acetic acid. It is freely soluble in acetonitrile and acetone and soluble in toluene. It is very slightly soluble in n-hexane.

Brivaracetam Oral Solution was launched in the United States on February 18, 2016. The original research is UCB INC, and the trade name is BRIVIACT.

Brivaracetam is unstable in aqueous solution and easily forms degradation products. It is necessary to use excipients with low toxicity at the lowest concentration. The use of small amounts of some excipients may be insufficient to reach the level of physicochemical stability. It is difficult to find excipients that are both non-toxic and provide effective stability even at low concentrations.

Oral solution of brivaracetam is known in the prior art. There still remains a need in the art to provide an improved an oral solution comprising brivaracetam. In the present invention, the oral solution is created to overcome the above problems and provides additional advantages to the relevant field of art. Other advantages and embodiments of the present invention will be clarified in the following description.

### Detailed Description of the Invention

The main object of the present invention is to provide a brivaracetam oral solution has simple preparation technique, convenient administration, quick effect, good taste which improves patient compliance.

Another object of the present invention is to solve the problem of oral liquid preparation technical, claims a preparation process of oral liquid safe taking and good stability,

In fact, active pharmaceutical agents in liquid forms are more susceptible to chemical and physical instability than in the solid state. In addition to the solubility, the active pharmaceutical agent needs to be physically and chemically stable in the oral solution. Trace amounts of impurities from active pharmaceutical agents or excipients and the pH of the solution may cause the degradation of the active pharmaceutical agent and this may cause an increase in instability when the solution is consistently introduced into the atmosphere.

Sweeteners that do not interact with the active substance, are effective even in small amounts and do not cause color change in the final product were used. In this way, it helped to ensure both physical and chemical stability. Also, it made the product both transparent in appearance and provided an easy-to-drink product for the patient compliance.

According to an embodiment of the present invention, an oral solution composition comprising brivaracetam and at least one sweetening agent is sodium cyclamate, acesulfame potassium or mixtures thereof.

According to an embodiment of the present invention, the sweetening agent is sodium cyclamate.

According to an embodiment of the present invention, the amount of sodium cyclamate is 0.15% to 1.0% by weight in the total composition.

According to an embodiment of the present invention, the sweetening agent is acesulfame potassium.

According to an embodiment of the present invention, the amount of acesulfame potassium is 3.0% to 4.2% by weight in the total composition.

According to an embodiment of the present invention, the amount of the sweetening agent is 0.15% to 4.2% by weight in the total composition.

According to an embodiment of the present invention, the oral composition further comprising sorbitol solution (70%) as the sweetening agent.

When we were preparing the composition, we saw that when used in the particle size ranges specified below help to provide stability. In addition, the process steps were faster and easier at these particle sizes. Clarity and homogeneity in the oral solution were at the desired level.

As used here in, 'particle size' means the cumulative volume size distribution as tested by any conventionally accepted method such as the laser diffraction method (i.e. Malvern analysis). The term d (0.1) means, the size at which 10% by volume of the particles are finer and d (0.5) means the size at which 50% by volume of the particles are finer and d (0.9) means the size at which %90 by volume of the particles is finer.

According to an embodiment of the present invention, an oral solution composition comprising brivaracetam wherein brivaracetam has a d (0.9) particle size between 50 µm to 250 µm.

According to one embodiment of this invention, brivaracetam has a d (0.9) particle size between 55 µm to 63 µm or between 64 µm to 72 µm or between 73 µm to 85 µm or between 86 µm to 97 µm or between 98 µm to 113 µm or between 115 µm to 127 µm or between 128 µm to 142 µm or between 143 µm to 154 µm or between 155 µm to 168 µm or between 170 µm to 184 µm or between 185 µm to 196 µm or between 198 µm to 217 µm or between 218 µm to 229 µm or between 230 µm to 245 µm.

According to one embodiment of this invention, brivaracetam has a d (0.1) particle size between 1 µm to 40 µm or between 8 µm to 34 µm or between 16 µm to 45 µm.

According to one embodiment of this invention, brivaracetam has a d (0.5) particle size between 25 µm to 85 µm or between 33 µm to 76 µm or between 40 µm to 70.

According to one embodiment of this invention, brivaracetam has a d (0.1) particle size between 1 µm .to 40 µm and a d (0.5) particle size between 8 µm to 85 µm and a d (0.9) particle size between 100 µm to 300 µm.

According to an embodiment of the present invention, the amount of brivaracetam is 0.1% to 7.0% by weight in the total composition, preferably 0.2% to 4.0% by weight in the total composition, preferably 0.3% to 1.8% by weight in the total composition.

According to an embodiment of the present invention, Brivaracetam is 10mg/ml in the oral solution.

According to an embodiment of the present invention, the composition further comprises at least one pharmaceutically acceptable excipient which is selected from the group comprising pH adjusters, antimicrobial agents, viscosity agents, sweetening agents, tonicity adjusting agents, flavoring agents or mixtures thereof.

Suitable pH adjuster agent are selected from the group comprising malic acid, sodium citrate, anhydrous citric acid, tartaric acid, sodium potassium tartrate, aluminum potassium sulfate, anhydrous disodium hydrogen phosphate, potassium carbonate, anhydrous sodium dihydrogen phosphate, dibasic potassium sulfate, calcium carbonate, nicotinic acid, dilute hydrochloric acid, glacial acetic acid, lactic acid, maleic acid, monobasic potassium phosphate, phosphoric acid, adipic acid, sodium acetate, sodium bicarbonate, sodium carbonate, sodium dihydrogen phosphate dihydrate, tribasic sodium phosphate or mixtures thereof.

According to one embodiment of this invention, the pH adjuster agent is sodium citrate, anhydrous citric acid or mixtures thereof.

Suitable antimicrobial agents are selected from the group comprising methyl paraben, propyl paraben, benzyl alcohol, benzoic acid, boric acid, sorbic acid and their salts thereof, benzalkonium chloride or mixtures thereof.

According to one embodiment of this invention, the antimicrobial agent is methyl paraben, propyl paraben or mixtures thereof.

Suitable viscosity agents are selected from the group comprising microcrystalline cellulose, sodium carboxymethyl cellulose, carbomer, liquid paraffin, polyacrylamide, hydroxyethyl cellulose, methyl cellulose, hydroxypropyl methyl cellulose, hydroxypropyl cellulose, guar gum, xanthan gum, acacia, and tragacanth, modified starch, acrylic acid/ethyl acrylate copolymers, polymethacrylate copolymers, maleic anhydride-alkyl methylvinylethers and copolymers, aluminum magnesium hydroxy stearate, poloxamer, polyvinyl alcohol, polyvinylpyrrolidone, ethyl hydroxybenzoate, propyl hydroxybenzoate, butyl hydroxybenzoate or mixtures thereof.

According to one embodiment of this invention, the viscosity agent is sodium carboxymethyl cellulose.

Suitable tonicity adjusting agents is selected from the group comprising glycerin, sodium chloride, potassium chloride, calcium chloride, mannitol, glycerol, sorbitol, propylene glycol, dextrose, sucrose or mixtures thereof.

According to one embodiment of this invention, the tonicity adjusting agent is glycerin.

Suitable flavoring agents are selected from the group comprising menthol, peppermint, cinnamon, chocolate, vanillin or fruit essences such as cherry, orange, strawberry, grape, black currant, raspberry, banana, red fruits, wild berries or mixtures thereof.

According to one embodiment of this invention, the flavoring agent is raspberry, strawberry or mixtures thereof.

Suitable solvents are selected from the group comprising pure water, propylene glycol, ethanol, polyethylene glycol or mixtures thereof.

According to one embodiment of this invention, the oral solution composition comprises;
- Brivaracetam
- Sodium citrate
- Anhydrous citric acid
- Methylparaben
- Sodium carboxymethylcellulose
- Acesulfame potassium or sodium cyclamate
- Sorbitol solution 70%

In the present invention, a process operation is simple, and it is easy to prepare, good repeatability of preparation sample, for producing cost is not high.

A process for the preparation of the oral solution composition comprising brivaracetam comprises the following steps:
a) Sodium citrate, anhydrous citric acid, methyl paraben, acesulfame potassium or sodium cyclamate, and brivaracetam is dissolved in water,
b) Sodium carboxymethyl cellulose is added and dissolved in solution obtained in step-a,
c) Sorbitol solution added and mixed,
d) Glycerin added and mixed,
e) Rasberry flavor is added and mixed.

### Example 1: An oral solution composition

| **Ingredients** | **% by weight** |
|---|---|
| Brivaracetam | 0.89 |
| Sodium citrate | 0.26 |
| Anhydrous citric acid | 0.08 |
| Methylparaben | 0.09 |
| Sodium carboxymethylcellulose | 0.45 |
| Acesulfame potassium | 3.58 |
| Sorbitol solution 70% | 30.70 |
| Glycerin | 13.60 |
| Flavor | 0.50 |
| Solvent | 49.85 |
| **Total** | **100** |

A process for example 1;
a) Sodium citrate, anhydrous citric acid, methyl paraben, acesulfame potassium and brivaracetam is dissolved in water,
b) Sodium carboxymethyl cellulose is added and dissolved in solution obtained in step-a,
c) Sorbitol solution added and mixed,
d) Glycerin added and mixed,
e) Rasberry flavor is added and mixed.

### Example 2: An oral solution composition

| **Ingredients** | **% by weight** |
|---|---|
| Brivaracetam | 0.89 |
| Sodium citrate | 0.26 |
| Anhydrous citric acid | 0.08 |
| Methylparaben | 0.09 |
| Sodium carboxymethylcellulose | 0.45 |
| Sodium cyclamate | 0.17 |
| Sorbitol solution 70% | 30.70 |
| Glycerin | 13.60 |
| Flavor | 0.50 |
| Solvent | 53.26 |
| **Total** | **100** |

A process for example 2;
a) Sodium citrate, anhydrous citric acid, methyl paraben, sodium cyclamate and brivaracetam is dissolved in water,
b) Sodium carboxymethyl cellulose is added and dissolved in solution obtained in step-a,
c) Sorbitol solution added and mixed,
d) Glycerin added and mixed,
e) Rasberry flavor is added and mixed.

## Claims

1. An oral solution composition comprising brivaracetam and at least one sweetening agent is sodium cyclamate, acesulfame potassium or mixtures thereof.

2. The oral solution composition according to claim 1, wherein the sweetening agent is sodium cyclamate.

3. The oral solution composition according to claim 2, wherein the amount of sodium cyclamate is 0.15% to 1.0% by weight in the total composition.

4. The oral solution composition according to claim 1, wherein the sweetening agent is acesulfame potassium.

5. The oral solution composition according to claim 4, wherein the amount of acesulfame potassium is 3.0% to 4.2% by weight in the total composition.

6. The oral solution composition according to claim 1, wherein the amount of the sweetening agent is 0.15% to 4.2% by weight in the total composition.

7. The oral solution composition according to claim 1, wherein the oral composition further comprising sorbitol solution (70%) as the sweetening agent.

8. The oral solution composition according to claim 1, wherein brivaracetam has a d (0.9) particle size between 50 µm to 250 µm.

9. The oral solution composition according to claim 1, wherein the amount of brivaracetam is 0.1% to 7.0% by weight in the total composition, preferably 0.2% to 4.0% by weight in the total composition, preferably 0.3% to 1.8% by weight in the total composition.

10. The oral solution composition according to claim 1, wherein the composition further comprises at least one pharmaceutically acceptable excipient which is selected from the group comprising pH adjusters, antimicrobial agents, viscosity agents, tonicity adjusting agents, flavoring agents or mixtures thereof.

11. The oral solution composition according to claim 1, wherein the oral solution composition comprises;
• Brivaracetam
• Sodium citrate
• Anhydrous citric acid
• Methylparaben
• Sodium carboxymethylcellulose
• Acesulfame potassium or sodium cyclamate
• Sorbitol solution 70%
